# EUROPEAN PATENT SPECIFICATION

(11) **EP 3 960 125 B1**
(45) Date of publication and mention of the grant of the patent: **26.11.2025**
(21) Application number: 20795245.8
(22) Date of filing: 22.04.2020
(51) Int. Cl.: A61F 2/16

(54) **ACCOMMODATING INTRAOCULAR LENS**
AKKOMMODIERENDE INTRAOKULARLINSE
LENTILLE INTRAOCULAIRE D'ACCOMMODATION

(30) Priority: 24.04.2019 JP 2019083030
(43) Date of publication of application: 02.03.2022
(73) Proprietor: Mirai Eye Inc., Hyogo 663-8113 (JP)
(72) Inventor: AKURA, Junsuke, Nishinomiya-shi, Hyogo 663-8113 (JP)
(74) Representative: Viering, Jentschura & Partner mbB Patent- und Rechtsanwälte
(86) International application number: PCT/JP2020/017307
(87) International publication number: WO 2020/218327

(56) References cited:
- WO-A1-2018/015255
- WO-A1-2019/069948
- WO-A1-2019/069948
- JP-A- 2018 538 060
- US-A1- 2005 021 139
- US-A1- 2011 071 628

## Description

### Technical Field

The present invention relates to an accommodating intraocular lens to be inserted into a lens capsule whose anterior capsule has been incised in ophthalmic surgery, such as, e.g., lens capsule extraction surgery to be performed as cataract surgery, refractive surgery, or presbyopia correction surgery.

### Background of the Invention

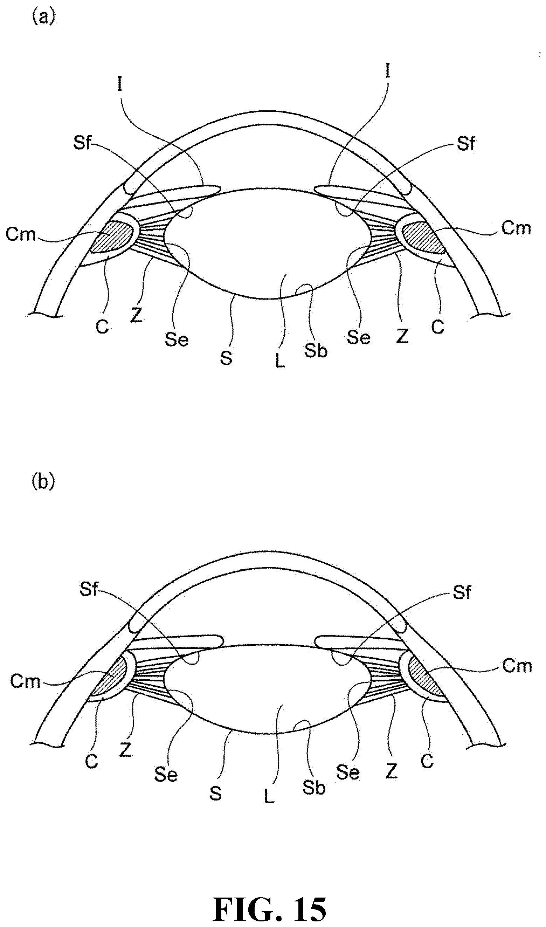

Usually, focus adjustment of a human eye is performed by changing a thickness of a crystalline lens. A crystalline lens L is a convex transparent lens with a diameter of about 9 mm to 11 mm and a thickness of about 4 mm to 5 mm, as shown in FIG. 15. The crystalline lens L is fixed to the ciliary body C via the Zinn's zonule Z in a state of being wrapped by a transparent lens capsule S behind the iris I. The crystalline lens L adjusts the focal point mainly by changing the curvature of the anterior surface of the crystalline lens L in accordance with the movement of the ciliary body C at the time of the focus adjustment.

A specific adjustment mechanism will be explained. For example, when looking at the distance, as shown in (b) of FIG. 15, the ciliary muscle Cm of the ciliary body C is relaxed, and the ciliary body C is in a retracted position away from the lens capsule S. In this condition, a relatively strong tension is generated in the Zinn's zonule Z positioned between the ciliary body C and the equator Se of the lens capsule S. With this, the equator Se of the lens capsule is pulled radially outward. With this, the curvature of the anterior surface of the crystalline lens L in the lens capsule S is reduced to perform the focus adjustment at the time of the distance vision.

On the other hand, when adjustment effort is made to see a nearby object, as shown in (a) of FIG. 15, the ciliary muscle Cm of the ciliary body C contracts to cause the ciliary body C to protrude centripetally (in the direction toward the lens capsule equatorial portion Se), so that the ciliary body C is positioned in the direction closer to the lens capsule S. This weakens the tension of the Zinn's zonule Z. For this reason, the curvature of the anterior surface increases due to the elasticity inherent in the crystalline lens L, thereby performing the focus adjustment at the time of the near vision.

As the ciliary muscle Cm of the ciliary body C contracts and relaxes as described above, the focus adjustment is performed mainly by changing the curvature of the anterior surface of the crystalline lens L to refract the light incident on the eye. Note that in this adjustment mechanism, it is known that the contraction function and the relaxation function by the ciliary muscle Cm of the ciliary body C are kept relatively well even we get older. On the other hand, the cortices and the nuclei, which are the contents of the crystalline lens L, harden as we get older to lose flexibility, causing the curvature of the anterior surface of the crystalline lens L to become less likely to be changed. For this reason, it is known that the ability to freely adjust the focal point from the distance vision state to the near vision state is lost (this is called "presbyopia" ).

Incidentally, among diseases occurring in the above-described crystalline lens L, there is a disease called "cataract" in which the crystalline lens becomes cloudy mainly due to aging. Many individuals have cataract surgery to treat the cataract. This surgery normally applies a method in which a circular hole is formed in the anterior capsule Sf, the turbid contents of the crystalline lens L are extracted via the hole by phacoemulsification, and an intraocular lens is inserted into the lens capsule S in a state in which only the transparent lens capsule S in the incised state remains. Cataract surgery by this method has been currently performed to over one million patients per year in Japan and over three million patients per year in the United States of America. A variety of intraocular lenses to be used for the surgery has been proposed.

For example, the intraocular lens described in Patent Document 1 is a so-called accommodating intraocular lens. This intraocular lens is composed of an optical portion (optical lens 42) and a support portion (optical lens positioning component 46). The support portion is provided with an anterior portion, a posterior portion, and a curved portion connecting the anterior portion and the posterior portion. The optical portion and the anterior portion of the support portion are connected via haptic arms. With this, the support portion deforms in response to the movement of the lens capsule when in the distance vision state and in the near vision state to allow the optical portion to move in the anterior-posterior direction (see Patent Document 1). Patent Document 2 discloses an elastomeric intraocular adaptive optics that adapts (i.e., accommodates and disaccommodates) in response to normal physiologic zonular de-tensioning and tensioning forces. Patent Document 3 discloses an accommodative intraocular lens capable of effectively achieving a focus adjustment function. It comprises an optical part, and a plurality of support parts that are positioned at the periphery of the optical part. The support parts comprise a front support section and a rear support section, and due to the elasticity of the support parts, the front support section presses an anterior capsule, while the rear support section presses a posteriori capsule. When a lens capsule is in a distance vision or near vision state, if the pressure of the anterior capsule on the front support section increases or decreases, the front support section maintains the position of a base end section in a radial direction while bending to the rear and returning to the front, thus causing a tip end section of the front support section to move considerably rearward or forward while maintaining a constant position in the radial direction, and in accordance with this movement, the optical part moves considerably backward or forward. Patent Document 4 discloses an arrangement for an artificial eye lens. The arrangement comprises at least two ring-shaped elements and a plurality of arched ribs. The plurality of arched ribs connects the two ring-shaped elements. The ring-shaped elements and plurality of arched ribs form a space for housing the artificial eye lens. The ring-shaped elements are moveable with respect to each other along a common axis.

### Prior Art Document

### Patent Document

Patent Document 1: Japanese Translation of PCT International Application Publication No. 2006-503661; Patent Document 2: US 2005/0021139A1; Patent Document 3: WO 2019/069948 A1; Patent Document D4: WO 2018/015255 A1

### SUMMARY OF THE INVENTION

### Problems to be Solved by the Invention

However, the above-described accommodating intraocular lens is merely configured such that the support portion (particularly, the curved portion) is simply expanded or narrowed in the radial direction in response to the movement of the lens capsule. For this reason, only slight focus adjustment functions can be exhibited, and therefore it was difficult to exert practical focus adjustment functions enabling from reading to driving without using glasses. For this reason, there has been a great need for an accommodating intraocular lens capable of capturing minute movements of the lens capsule caused by weak forces of shrinkage and relaxation of the ciliary muscle and amplifying the movements into relatively large movements of an optical portion to thereby exert practical focus adjustment functions.

This will be explained in more detail. A lens capsule of a physiologically normal crystalline lens merely moves about up to 0.4 mm at the most actively moving portion (the central portion of the anterior capsule). It is known that the anterior capsule is incised in a circular shape with a diameter of about 5 mm by surgery and that the remaining lens capsule close to the equator of the crystalline lens moves at best by about 0.25 mm. Thus, simple movements of the optical portion of the accommodating intraocular lens in accordance with the movements of the lens capsule generates only weaker adjustment ability.

For example, according to the calculation by a ray-tracing method by replacing the adjustment ability with a refractive power of 22 diopters, which is the typical refractive power of an accommodating intraocular lens, it can be seen that only an adjustment ability of 0.5 diopters is produced when an optical portion of an accommodating intraocular lens is moved by 0.25 mm.

To perform focus adjustments covering from 33 cm distance suitable for reading to infinity, accommodating power of 3 diopters is required. On the other hand, it is known that a human eye has a pseudo-accommodative force of about 2 diopters due to pinhole effects caused by pupil constriction and multifocal properties of a cornea. For this reason, it is enough that an accommodating intraocular lens generates the focus adjustment ability of one diopter. Adding one diopter of the accommodating intraocular lens to two diopters of the pseudo-accommodative force yields three diopters. Thus, it is possible to obtain a practical minimum focus adjustment ability covering from reading to operating a personal computer or driving a vehicle.

When a movement of an optical portion of an accommodating intraocular lens required for the accommodating intraocular lens having a typically refractive power of 22 diopters to produce an adjustment ability of one diopter or greater is calculated by a ray-tracing method using a Gullstrand model eye, it can be confirmed that the optical portion must move more than 0.5 mm.

Therefore, in order for the accommodating intraocular lens to exert a practical focus adjustment ability, it is required to have some amplifying function of capturing the lens capsule' s minute movements of about 0.25 mm and amplifying it to the optical portion movement of 0.5 mm or more.

On the other hand, a crystalline lens is a convex lens having a thickness of 4 mm to 5 mm and a diameter of 9 mm to 11 mm, the convex lens being packed with elastic cortices and nuclei in a thin lens capsule having a thickness of 10 µm to 20 µm. In a cataract surgery or the like, an anterior lens capsule of a crystalline lens is circularly incised to remove the crystalline lens in the lens capsule, and then an intraocular lens is placed therein. However, after the surgery, the lens capsule collapses, flattens, and loses the tension, resulting in adhesion to the lens capsule (particularly to the equator). As a result, the lens capsule cure due to lens epithelial cell proliferation and fibrosis may occur, and in some cases, a secondary cataract may occur.

The present invention has been made in view of the technical background described above. It is an object of the present invention to provide an accommodating intraocular lens firstly capable of capturing minute movements of a lens capsule and amplify them to large movements of an optical portion, and secondly capable of reliably preventing the lens capsule from being hardened due to adhesion of the lens capsule followed by growth and fibrosis of lens epithelial cells, which in turn can exert a practical focus adjustment function.

### Means for Solving the Problem

An accommodating intraocular lens according to the present invention has the following configuration in order to achieve the above-described object. That is, the accommodating intraocular lens is composed of a lens capsule expansion bag to be placed in a lens capsule whose anterior capsule has been incised in ophthalmic surgery, and an intraocular lens to be placed inside the lens capsule expansion bag. The the lens capsule expansion bag has an elastic force. The lens capsule expansion bag includes: an annular anterior bag portion to be placed in such a manner as to be in contact with an anterior capsule of the crystalline lens; an annular posterior bag portion to be placed in such a manner as to be in contact with a posterior capsule of lens capsule; and an annular intermediate bag portion protruding radially outward from an outer peripheral portion of the anterior bag portion and an outer peripheral portion of the posterior bag portion, the annular intermediate bag portion being configured to be placed in such a manner as to be in contact with an equator of the lens capsule. The intraocular lens includes: an optical portion; and one or a plurality of support portions arranged around the optical portion to support the optical portion. The support portion has an elastic force. The support portion includes: an anterior support portion to be placed in such a manner as to be in contact with an inner surface of the anterior bag portion; and a posterior support portion to be placed in such a manner as to be in contact with an inner surface of the posterior bag portion. The anterior support portion extends radially inward and forward from a base end portion connected to the posterior support portion and then extends radially inward and rearward, an anterior end portion of the anterior support portion being connected to a peripheral portion of the optical portion. When the lens capsule is in a distance vision state, as a pressing force by the anterior bag portion to the anterior support portion of the intraocular lens increases in accordance with an increase in a pressing force of the lens capsule expansion bag by the anterior capsule to the anterior support portion of the lens capsule expansion bag, the anterior support portion deflects radially inward and rearward while maintaining a radial position of the base end portion and a radial position of the anterior end portion, causing a rearward movement of the anterior end portion of the anterior support portion while maintaining the radial position of the anterior end portion of the anterior support portion, which moves the optical portion rearward, and the intermediate bag portion of the lens capsule expansion bag deforms to expand radially outward in response to a movement of the equator of the lens capsule while contacting the equator of the lens capsule. When the lens capsule is in a near vision state, as a pressing force by the anterior bag portion to the anterior support portion of the intraocular lens decreases in accordance with a decrease in a pressing force of the lens capsule expansion bag by the anterior capsule to the anterior support portion of the lens capsule expansion bag, the anterior support portion deflects radially outward and forward while maintaining a radial position of the base end portion and a radial position of the anterior end portion, causing a forward movement of the anterior end portion of the anterior support portion while maintaining the radial position of the anterior end portion of the anterior support portion, which moves the optical portion forward, and the intermediate bag portion of the lens capsule expansion bag deforms to contract radially inward in response to a movement of the equator of the lens capsule while contacting the equator of the lens capsule.

According to this, when the lens capsule is in a distance vision state, as the pressing force by the anterior capsule to the anterior bag portion of the lens capsule expansion bag increases in accordance with the increase in the pressing force by the anterior capsule to the anterior support portion of the intraocular lens, the anterior support portion largely deflects radially inward and rearward. With this, the anterior end portion of the anterior support portion moves largely rearward than the movement of the anterior capsule, which enables the large rearward movement of the optical portion. On the other hand, when the lens capsule is in the near vision state, as the pressing force by the anterior bag portion to the anterior support portion decreases in accordance with the decrease in the pressing force to the anterior bag portion of the lens capsule expansion bag by the anterior capsule, the anterior support portion largely returns radially outward and forward to its original condition by the elastic force. With this, the anterior end portion of the anterior support portion moves forward more than the movement of the anterior capsule, which enables the large movement of the optical portion accordingly. For this reason, it is possible to capture the minute movements of the lens capsule and amplify them to large movements of the optical portion.

Further, when the lens capsule is in the distance vision state, the intermediate bag portion of the lens capsule expansion bag deforms to expand radially outward in response to the movement of the equator of the lens capsule while contacting the equator of the lens capsule. On the other hand, when the lens capsule is in the near vision state, the intermediate bag portion of the lens capsule expansion bag deforms to contract radially inward in response to the movement of the equator of the lens capsule while contacting the equator of the lens capsule. Therefore, it becomes a state in which there is always no gap between the lens capsule expansion bag and the equator of the lens capsule, and the equator of the lens capsule is always open in the anterior-posterior direction. Therefore, it is possible to assuredly prevent the adhesion of the lens capsule and the subsequent hardening of the lens capsule by growth and fibrosis of the lens epithelial cell.

The lens capsule expansion bag may be configured such that the intermediate portion presses the equator of the lens capsule. When the lens capsule is in the distance vision state, the intermediate bag portion may deform to expand radially outward in response to the movement of the equator of the lens capsule while pressing the equator of the lens capsule. On the other hand, when the lens capsule is in the near vision state, the intermediate bag portion may deform to contract radially inward in response to the movement of the equator of the lens capsule while pressing the equator of the lens capsule. With this, it becomes a state in which there is always no gap between the lens capsule expansion bag and the equator of the lens capsule and that the equator of the lens capsule is open in the anterior-posterior direction more assuredly. Therefore, it is possible to more assuredly prevent the adhesion of the lens capsule and the subsequent hardening of the lens capsule due to proliferation and fibrosis of the lens epithelial cell.

Further, the lens capsule expansion bag may be configured such that the anterior bag portion, the posterior bag portion, and the intermediate bag portion are continuously formed of a flexible membrane-like or plate-like elastic member. According to this, the lens capsule expansion bag can be easily molded, and the anterior bag portion, the posterior bag portion, and the intermediate bag portion can be smoothly deformed in accordance with the movements of the lens capsule.

Further, an inner peripheral portion of at least one of the anterior bag portion and the posterior bag portion of the lens capsule expansion bag may be formed to be thicker than a portion of the lens capsule expansion bag other than the inner peripheral portion. This prevents the unintentional radial deformation of the inner peripheral portion of the anterior bag portion and the inner peripheral portion of the posterior bag portion when the anterior bag portion and the posterior bag portion entirely deflect in response to the movements of the lens capsule. This allows the anterior bag portion and the posterior bag portion to largely deflect in response to small movements of the lens capsule.

Further, the lens capsule expansion bag may be configured such that the intermediate bag portion is smaller in rigidity than the anterior bag portion and the posterior bag portion. This makes it easier for the intermediate bag portion to deform in accordance with the movements of the equator of the lens capsule.

Further, the lens capsule expansion bag may be provided with a plurality of openings formed in a side surface of the intermediate bag portion at predetermined intervals. This makes it easier for the intermediate bag portion to deform in accordance with the movements of the equator of the lens capsule.

Further, the lens capsule expansion bag may be coated with an MPC-polymer on at least one of an outer surface and a back surface. Further, the lens capsule expansion bag may be formed of a gel-like material with lubricity. This configuration allows the biocompatibility of the lens capsule expansion bag and the lens capsule to be improved and allows the lens capsule expansion bag to smoothly deform in accordance with the movements of the lens capsule or allows the intraocular lens to smoothly deform in accordance with the movements of the lens capsule expansion bag.

Further, the anterior bag portion is composed of a plurality of anterior bag pieces arranged along a circumferential direction, and the adjacent anterior bag pieces are connected in a circumferential direction by a deformable connecting portion. Preferably, the connecting portion is formed in a mountain shape protruding radially inward or radially outward of the lens capsule expansion bag. With this configuration, it becomes easier for the anterior bag portion to deform backward and rearward, which allows the change in the pressing force to the anterior bag portion of the lens capsule expansion bag due to the anterior capsule to be accurately transmitted by the anterior support portion of the intraocular lens.

### Effects of the Invention

According to the present invention, when the lens capsule is in the distance vision state, as the pressing force by the anterior capsule by the anterior support portion of the intraocular lens increases in accordance with the increase in the pressing force by the lens capsule to the anterior bag portion of the lens capsule expansion bag, the anterior support portion significantly deflects radially inward and rearward. With this, the anterior end portion of the anterior support portion moves rearward more than the movement of the anterior capsule, which enables the optical portion to largely move backward. On the other hand, when the lens capsule is in the near vision state, as the pressing force by the anterior bag portion to the anterior support portion decreases in accordance with the decrease in the pressing force by the anterior capsule to portion of the anterior bag portion and an outer peripheral portion of the posterior bag portion, the annular intermediate bag portion being configured to be placed in such a manner as to be in contact with an equator of the lens capsule,
wherein when the lens capsule is in a distance vision state, the intermediate bag portion of the lens capsule expansion bag deforms to expand radially outward in response to a movement of the equator of the lens capsule while contacting the equator of the lens capsule, and
wherein when the lens capsule is in a near vision state, the intermediate bag portion of the lens capsule expansion bag deforms to contract radially inward in response to a movement of the equator of the lens capsule while contacting the equator of the lens capsule.

With this configuration, it becomes a state in which there is always no gap between the lens capsule and the equator of the lens capsule and that the equator of the lens capsule is more assuredly open in the anterior-posterior direction, which can more assuredly prevent hardening of the lens capsule due to adhesion of the lens capsule and subsequent lens epithelial cell growth and fibrosis.

### Effects of the Invention

According to the present invention, when the lens capsule is in the distance vision state, as the pressing force by the anterior capsule by the anterior support portion of the intraocular lens increases in accordance with the increase in the pressing force by the lens capsule to the anterior bag portion of the lens capsule expansion bag, the anterior support portion significantly deflects radially inward and rearward. With this, the anterior end portion of the anterior support portion moves rearward more than the movement of the anterior capsule, which enables the optical portion to largely move backward. On the other hand, when the lens capsule is in the near vision state, as the pressing force by the anterior bag portion to the anterior support portion decreases in accordance with the decrease in the pressing force by the anterior capsule to the anterior bag portion of the lens capsule expansion bag, the anterior support portion returns to its original condition radially outward and forward by the elastic force. This allows the anterior end portion of the anterior support portion to significantly move forward by more than the movement of the anterior capsule, which enables the optical portion to move forward accordingly. For this reason, it is possible to capture the minute movements of the lens capsule and amplify them to large movements of the optical portion.

Further, when the lens capsule is in the distance vision state, the intermediate bag portion of the lens capsule expansion bag deforms so as to expand radially outward in accordance with the movement of the equator of the lens capsule while contacting the equator of the lens capsule. On the other hand, when the lens capsule is in the near vision state, the intermediate bag portion of the lens capsule expansion bag deforms so as to contract radially inward in accordance with the movement of the equator of the lens capsule while contacting the equator of the lens capsule. With this, it becomes a state in which there is always no gap between the lens capsule expansion bag and the equator of the lens capsule and that the equator of the lens capsule is always open in the anterior-posterior direction, which can assuredly prevent the adhesion of the lens capsule and the subsequent curing of the lens capsule due to the growth and the fibrosis of lens epithelial cells.

### BRIEF DESCRIPTION OF THE DRAWINGS

FIG. 1 is a perspective view of a lens capsule expansion bag of an accommodating intraocular lens according to a first embodiment.
FIG. 2 is a plan view of the lens capsule expansion bag of FIG. 1.
FIG. 3 is a bottom view of the lens capsule expansion bag of FIG. 1.
FIG. 4 is a side view of the lens capsule expansion bag of FIG. 1.
FIG. 5 is a cross-sectional view of the lens capsule expansion bag of FIG. 1.
FIG. 6 is a perspective view showing an intraocular lens of the accommodating intraocular lens according to this embodiment.
FIG. 7 is a plan view of the intraocular lens of FIG. 6.
FIG. 8 is a bottom view of the intraocular lens of FIG. 6.
FIG. 9 is a side view of the intraocular lens of FIG. 6.
FIG. 10 is a cross-sectional view of the intraocular lens of FIG. 6.
FIG. 11 is a cross-sectional view showing a state in which the intraocular lens is placed inside the lens capsule expansion bag.
FIG. 12 is a schematic cross-sectional view showing the near vision state and the distance vision state of the accommodating intraocular lens.
FIG. 13 is an enlarged cross-sectional view of the intermediate bag portion of the lens capsule expansion bag according to a modification.
(a) of FIG. 14 is a plan view of the lens capsule expansion bag of the accommodating intraocular lens according to a second embodiment and (b) of FIG. 14 is a side view thereof.
FIG. 15 is a schematic cross-sectional view showing the movements at the time of the focus adjustments in a human eye.

### EMBODIMENTS FOR CARRYING OUT THE INVENTION

### <First Embodiment>

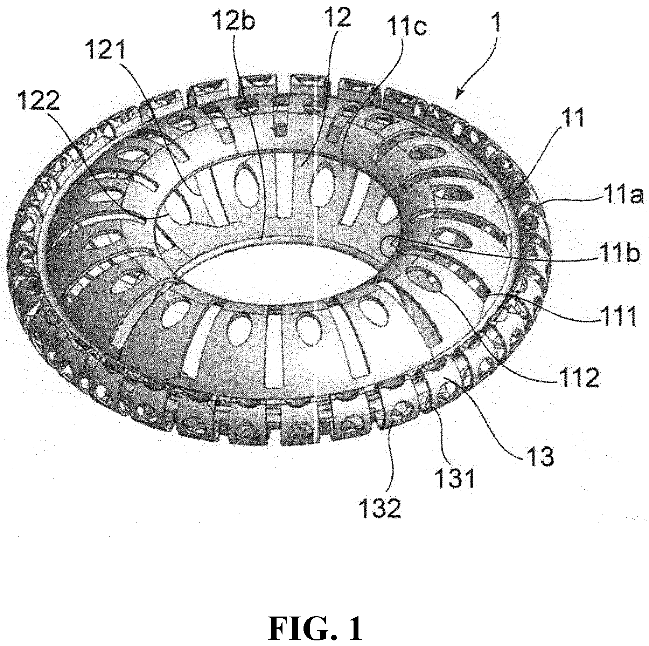

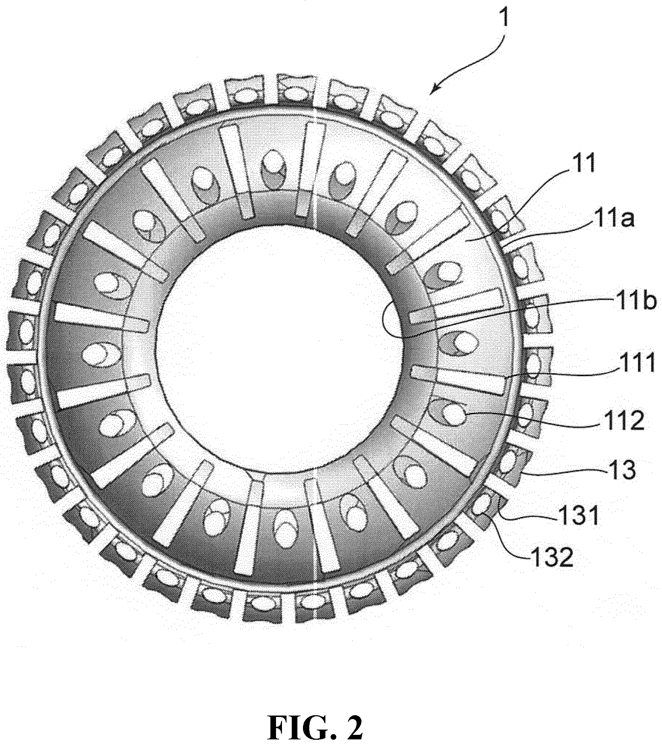

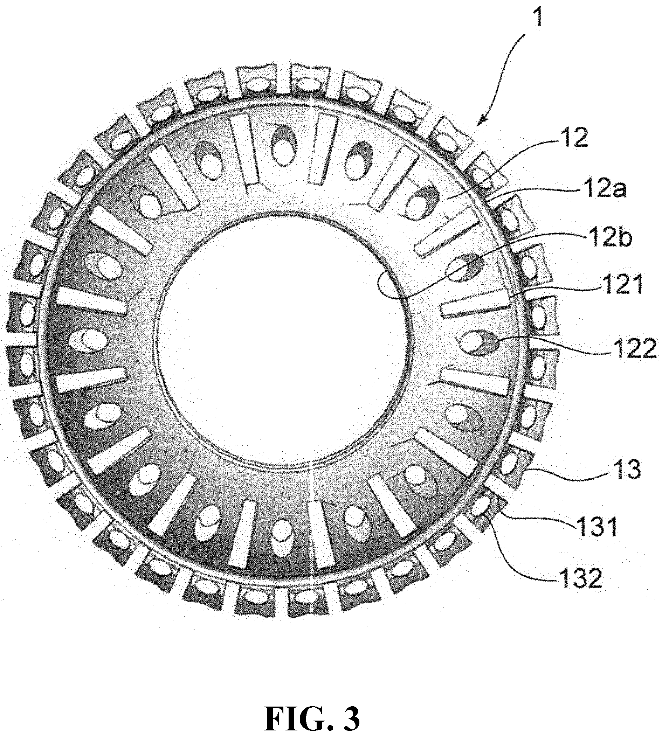

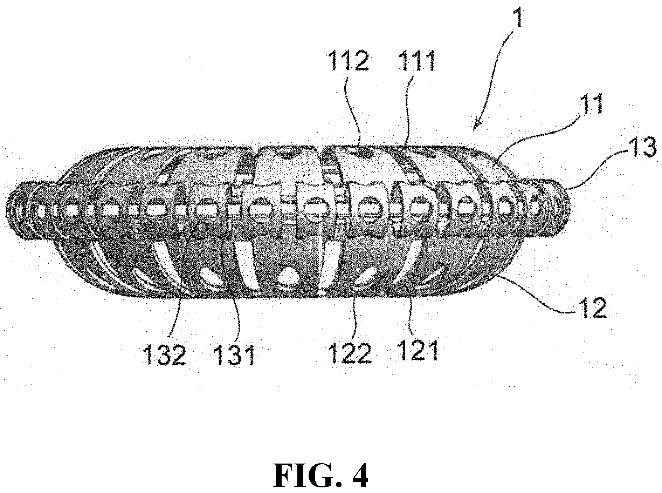

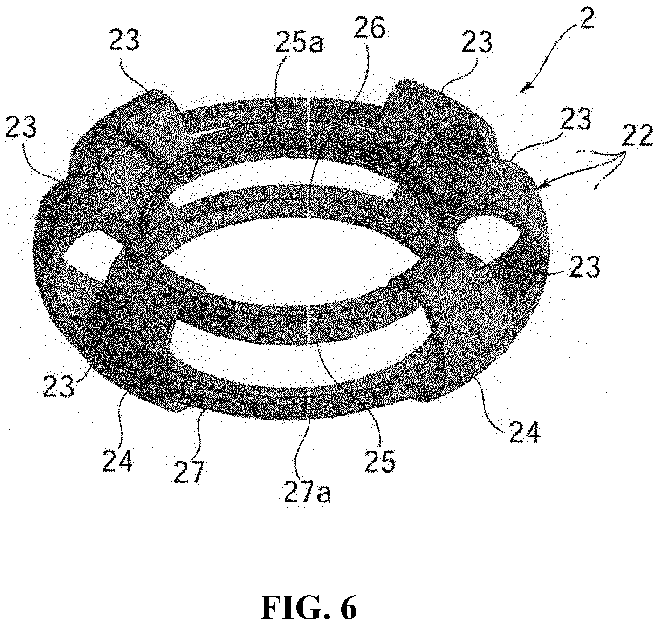

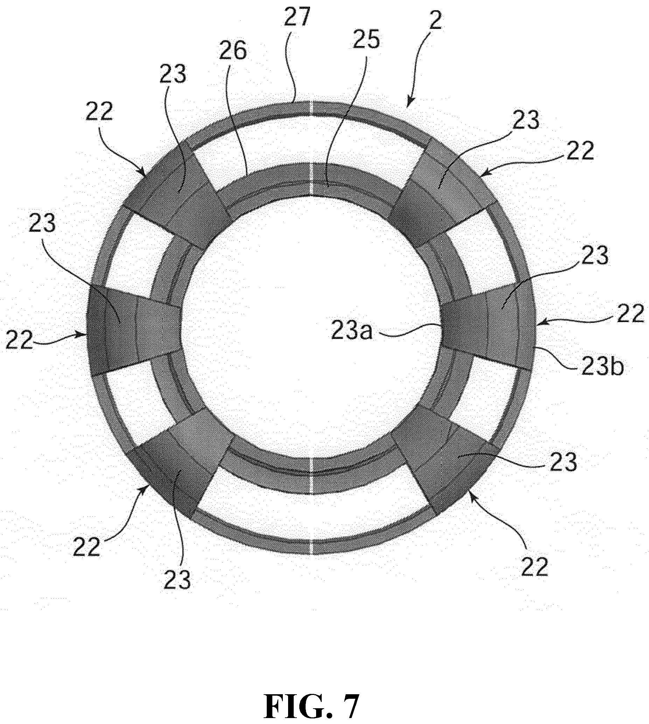

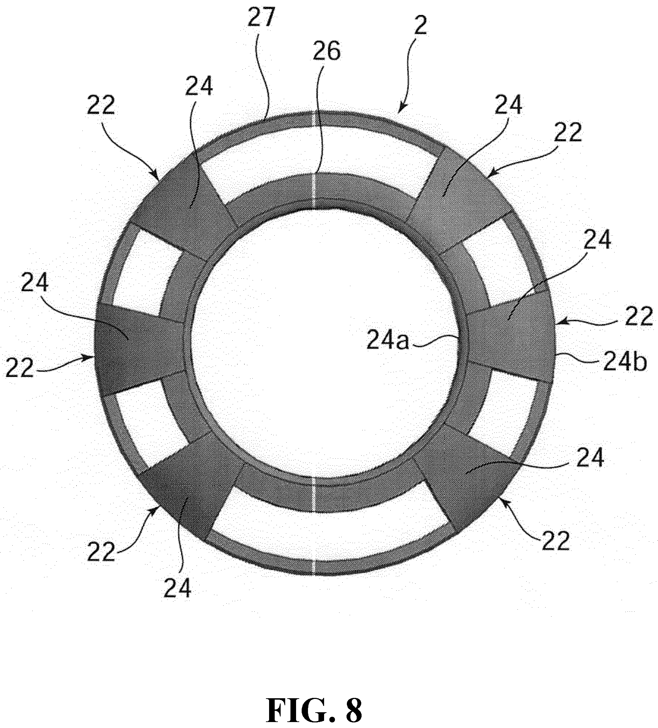

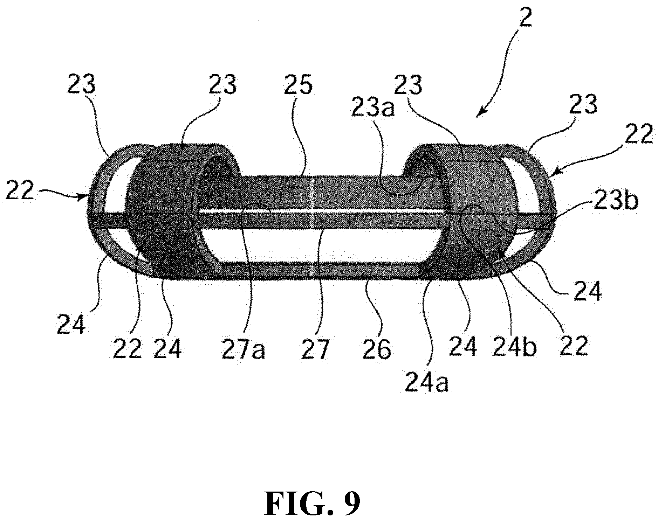

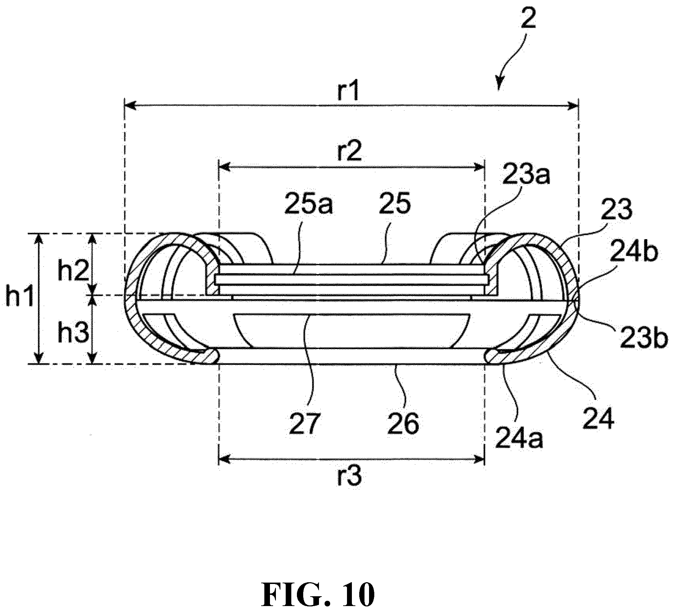

Next, a first embodiment of an accommodating intraocular lens according to the present invention will be described with reference to FIGS. 1 to 12. Note that the description will be made assuming that a direction toward the upper part in each drawing is an anterior direction of a human eye, and a direction toward the lower part of each drawing is a posterior direction of a human eye.

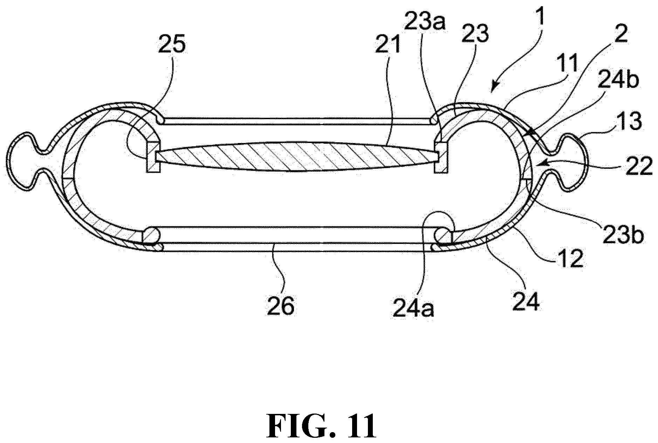

As shown in FIG. 11, this accommodating intraocular lens is composed of a lens capsule expansion bag 1 to be placed in a lens capsule S whose anterior capsule has been incised in ophthalmic surgery and an intraocular lens 2 to be placed in the lens capsule expansion bag 1.

As shown in FIGS. 1 to 5, the lens capsule expansion bag 1 is made of a flexible thin film-like or a plate-like elastic member (e.g., silicone member) and is formed in a hollow doughnut-like shape as a whole. An annular insertion opening 11c for inserting the intraocular lens 2 is formed along the circumferential direction at the inner peripheral portion of the lens capsule expansion bag 1.

Further, the lens capsule expansion bag 1 is provided with an annular anterior bag portion 11, an annular posterior bag portion 12, and an annular intermediate bag portion 13. The annular anterior bag portion 11 is placed in such a manner as to be in contact with the anterior capsule Sf of the lens capsule S. The annular posterior bag portion 12 is placed in such a manner as to be in contact with the posterior capsule Sb of the lens capsule S. The annular intermediate bag portion 13 protrudes radially outward from the outer peripheral portion of the anterior bag portion 11 and the outer peripheral portion of the posterior bag portion 12. This annular intermediate bag portion 13 is placed in such a manner as to be in contact with the equator Se of the lens capsule S. The anterior bag portion 11, the posterior bag portion 12, and the intermediate bag portion 13 are integrally molded by the above-mentioned elastic member.

The anterior bag portion 11 is formed in a shape such that it extends from the radially outward outer peripheral portion 11a while gradually curving radially inward and forward, then extends while gradually curving radially inward and rearward, and reaches the radially inward inner peripheral portion 11b. Further, the anterior bag portion 11 is formed to have a size of an outer diameter R1 of 9.6 mm, an inner diameter R2 of 5.0 mm, a height H1 of 1.2 mm, and a thickness (except for the inner peripheral portion) of 0.09 mm.

Further, in the anterior bag portion 11, a linear opening 111 extending in the radial direction and an oval opening 112 extending in the radial direction are arranged alternately along the circumferential direction. With this, the anterior bag portion 11 can be easily bent in accordance with the movements of the anterior capsule Sf of the lens capsule S, as will be described later.

The posterior bag portion 12 is formed such that it extends from the radially outward outer peripheral portion 12a while gradually curving radially inward and rearward and then reaches the radially inward inner peripheral portion 12b. Further, the posterior bag portion 12 is formed to have an outer diameter R1 of 9.6 mm, an inner diameter R3 of 5.0 mm, a height H2 of 1.7 mm, and a thickness (except for the inner peripheral portion) of 0.09 mm.

Further, in the posterior bag portion 12, a linear opening 121 extending in the radial direction and an oval opening 122 extending in the radial direction are formed alternately along the circumferential direction. With this, the posterior bag portion 12 can be easily bend in accordance with the movements of the posterior capsule Sb of the lens capsule S, as will be described later.

Further, the anterior bag portion 11 and the posterior bag portion 12 are each formed such that the thickness of the inner peripheral portion 11b, 12b is thicker than the thickness of a portion other than the inner peripheral portions 11b, 12b. As a result, the inner peripheral portions 11b, 12b are each configured as a ring-shaped member having rigidity larger than the rigidity of the portion other than the inner peripheral portions 11b, 12b. Therefore, when the anterior bag portion 11 and the posterior bag portion 12 entirely deflect in accordance with the movements of the lens capsule S as will be described later, it is prevented that the inner peripheral portions 11b and 12b of the anterior bag portion 11 and the posterior bag portion 12 inadvertently deform in the radial direction. Therefore, the anterior bag portion 11 and the posterior bag portion 12 can largely deflect in accordance with slight movements of the lens capsule S.

The intermediate bag portion 13 is formed to have a shape in such a manner as to extend from the outer peripheral portion 11a of the anterior bag portion 11 radially outward and forward while gently curving, then extend radially outward and rearward while gently curving as it is, and thereafter extend radially inward and rearward while gently curving and reach the outer peripheral portion 12b of the posterior bag portion 12. The intermediate bag portion 13 is formed to have a radial width W1 of 0.7 mm, a height H3 of 1.2 mm, and a thickness of 0.06 mm.

As described above, the intermediate bag portion 13 is formed to have a diameter substantially equal to or slightly larger than the diameter of the equator Se of the lens capsule S. As a result, when the intermediate bag portion 13 is placed in the lens capsule S, it slightly presses the equator Se of the lens capsule S. Therefore, it becomes a state that there is always no gap between the lens capsule expansion bag 1 and the equator Se of the lens capsule S and that the equator Se of the lens capsule S is more assuredly open in the anterior-posterior direction.

Further, the intermediate bag portion 13 is formed such that the thickness is thinner than the thickness of the anterior bag portion 11 and the thickness of the posterior bag portion 12 and that the rigidity is smaller than the rigidity of the anterior bag portion 11 and the rigidity of the posterior bag portion 12. As a result, the intermediate bag portion 13 can be easily deformed in accordance with the movements of the equator Se of the lens capsule S, as will be described later.

Further, the intermediate bag portion 13 is configured such that a linear opening 131 extending in the anterior-posterior direction and three circular openings 132 arranged in the anterior-posterior direction are alternately formed along the circumferential direction. With this configuration, the intermediate bag portion 13 can be easily deformed in accordance with the movements of the equator Se of the lens capsule S, as will be described later. Further, when these openings 131 and 132 are formed by laser, it is possible to reduce the cost and the delivery time.

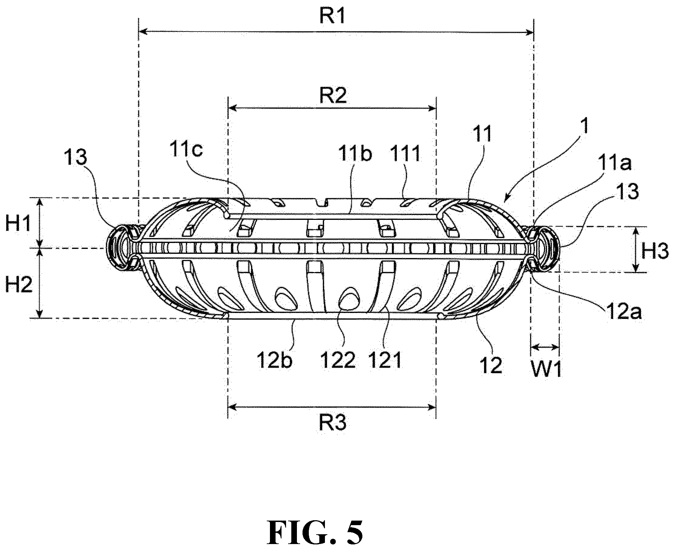

As shown in FIGS. 5 to 11, the intraocular lens 2 is provided with an optical portion 21, six support portions 22, an anterior ring portion 25, a posterior ring portion 26, and a restriction portion 27. The support portions 22 are arranged around the optical portion 21 to support the optical portion 21. The anterior ring portion 25 is provided on the anterior side of the respective support portions 22. The posterior ring portion 26 is provided on the posterior side of the respective support portion 22. The restriction portion 27 is provided at the intermediate portion of the support portion 22. The intraocular lens 2 is placed in the lens capsule expansion bag 1. Note that FIGS. 5 to 10 show the configuration of the intraocular lens 2 prior to the attachment of the optical portion.

The optical portion 21 is made of a synthetic resin material, such as, e.g., silicon, acryl, hydrogel, PMMA, HEMA, or hydro-polymer, and is a convex lens having a circular shape in plan view having a diameter of 5 mm to 6 mm. Note that the shape and the material of the optical portion 21 are not limited to these, and may be another shape and material.

The support portion 22 is composed of an anterior support portion 23 and a posterior support portion 24. The anterior support portion 23 is placed in such a manner as to be in contact with the inner surface of the anterior bag portion 11 of the lens capsule expansion bag 1. The posterior support portion 24 is placed in such a manner as to be in contact with the inner surface of the posterior bag portion 12 of the lens capsule expansion bag 1. Three pairs of anterior support portions 22 arranged in a radially opposed manner are arranged side by side.

The support portion 22 is formed to have a width in the circumferential direction of the intraocular lens 2 of 0.8 mm to 1.8 mm, a total height h1 of 2.55 mm in the anterior-posterior direction, an outer diameter r1 of 8.9 mm, an anterior inner diameter r2 of 5.2 mm, a posterior inner diameter r3 of 5.2 mm, and a thickness of 0.2 mm. The height h1 of the intraocular lens 2 is such a height that the intraocular lens 2 deflects slightly in the anterior-posterior direction when placed in the lens capsule expansion bag 1.

Further, the support portion 22 is made of a flexible material having a predetermined elastic force (e.g., a rubber hardness (A scale) of 20 degrees to 90 degrees, preferably 40 degrees to 70 degrees). The support portion 22 is configured such that the anterior support portion 23 deflects rearward in accordance with the movements of the anterior support portion 23 when the support portion 22 is placed in the lens capsule expansion bag 1. The concrete deflection movements of the support portion 22 will be described later, but it is said that the force of the ciliary muscle is 2 gf. Therefore, it is optimal that the reaction force is 0.5 gf to 5 gf when the portion where the anterior support portion 23 and the anterior capsule Sf are in contact is moved by 0.5 mm. This is expressed as a spring rate of 9.8 N/m to 98 N/m.

The anterior support portion 23 is formed in a shape such that it extends from the base end portion 23b connected to the posterior support portion 24 radially inward and forward while gently curving, then steeply curves, and extends radially inward and rearward while gently curving, and the anterior end portion 23a is connected to the anterior ring portion 25. The anterior support portion 23 has a height h2 (height from the outer peripheral portion to the bent portion) of 1.3 mm.

The posterior support portion 24 is formed in a shape such that it extends from the base end portion 24b connected to the anterior support portion 23 radially inward and rearward while gently curving and the posterior end portion 24a is connected to the posterior ring portion 26. This posterior support portion 24 is formed to have a height h3 (height from the inner peripheral portion to the outer peripheral portion) of 1.25 mm.

The anterior ring portion 25 is configured to connect the anterior end portions 23a of the adjacent anterior support portions 23 and hold the optical portion 21. The inner peripheral surface of the anterior ring portion 25 is provided with a groove 25a formed along the circumferential direction to hold the optical portion 21. The optical portion 21 is held by the anterior ring portion 25 with the peripheral portion fitted in the groove 25a.

The posterior ring portion 26 connects the posterior end portions 24a of the adjacent posterior support portions 24. The posterior ring portion 26 is stably placed on the inner surface of the posterior capsule Sb when placed in the lens capsule S.

Further, the restriction portion 27 is configured to connect the base end portions 24b of the adjacent posterior support portions 24 to regulate the radial movements of the anterior support portions 23 and the posterior support portions 24. With this, the anterior support portion 23 can deflect rearward and return forward in accordance with the movements of the lens capsule, as will be described later, in a state in which the radial position of the base end portion 23b of the anterior support portion 23 is maintained.

Note that the boundary portion between the anterior support portion 23 (base end portion 23b) and the posterior support portion 24 (base end portion 24b) refers to the radially outermost portion in the support portion 22 and refers to the height position of the anterior end portion 27a of the restriction portion 27 in this embodiment.

Next, a method of placing the intraocular lens 2 in a lens capsule S will be described.

In ophthalmic surgery, the lens capsule expansion bag 1 is inserted into a lens capsule S whose anterior capsule Sf has been incised, with an injector or tweezers. The lens capsule expansion bag 1 is placed such that the anterior bag portion 11 of the lens capsule expansion bag 1 is brought into contact with the inner surface of the anterior capsule Sf of the lens capsule S, the posterior bag portion 12 is brought into contact with the inner surface of the posterior capsule Sb of the lens capsule S, and the intermediate bag portion 13 is brought into contact with the inner surface of the equator Se of the lens capsule S.

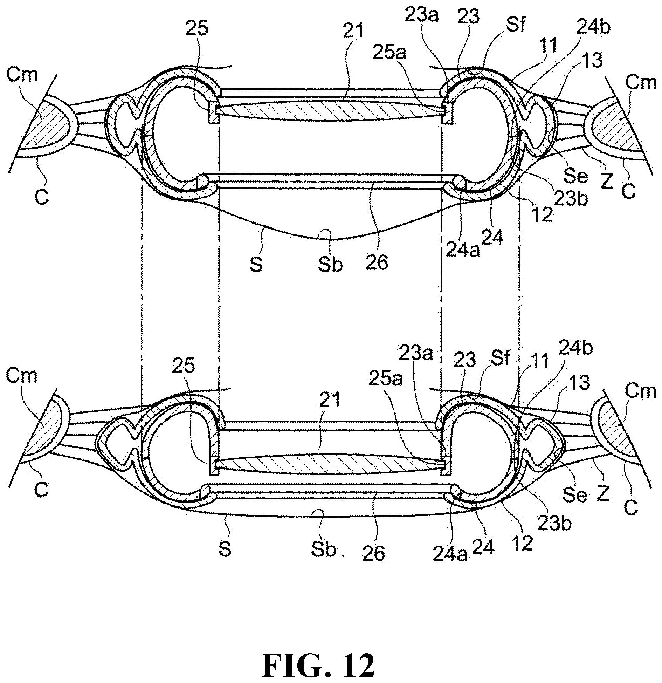

At this time, the lens capsule expansion bag 1 is formed such that the height in the anterior-posterior direction is slightly larger than the height of the lens capsule S in the anterior-posterior direction. Therefore, as shown in FIG. 12, the lens capsule expansion bag 1 becomes a state in which it is slightly deflected in the anterior-posterior direction in the lens capsule S. For this reason, by the elastic force of the lens capsule expansion bag 1, the anterior bag portion 11 comes into contact with the anterior capsule Sf so as to support the anterior capsule Sf, and the posterior bag portion 12 comes into contact with the posterior capsule Sb so as to support the posterior capsule Sb.

Next, the intraocular lens 2 is inserted into the lens capsule expansion bag 1 placed in the lens capsule S with an injector or tweezers. This insertion is performed such that the anterior support portion 23 (anterior capsule contact portion) of the intraocular lens 2 is brought into contact with the inner surface of the anterior bag portion 11 of the lens capsule expansion bag 1 and that the posterior support portion 24 is brought into contact with the inner surface of the posterior bag portion 12 of the lens capsule expansion bag 1. Further, the optical portion 21 is placed so as to be perpendicular to the anterior-posterior direction at the height position near the equator Se of the lens capsule S.

The equator Se of the lens capsule S expands so as to extend and expand the peripheral part of the equator Se of the lens capsule S in the anterior-posterior direction, and at the same time, the equator Se of the lens capsule S moves radially inward centripetally, and the diameter of the equator Se of the lens capsule S contracts. As a result, the Zinn's zonule Z is pulled in both directions toward the lens capsule S side and toward the ciliary body C side, and therefore tension is continuously applied to the Zinn's zonule Z. As a result, the tension is applied to the lens capsule S. Thus, the Zinn's zonule Z can transmit the slight contractions and relaxations of the ciliary muscle Cm of the ciliary body C to the lens capsule S.

Next, referring to FIG. 12, the operation of the intraocular lens 2 in the distance vision state and in the near vision state will be described.

In FIG. 12, the upper figure shows a vertical cross-sectional view showing the near vision state of the intraocular lens 2, and the lower figure shows a vertical cross-sectional view showing the distance vision state of the intraocular lens 2. Further, the one-dot chain lines in the upper and lower figures of FIG. 12 show that the radial positions of the outer peripheral portion and the inner peripheral portion of the anterior support portion 23 coincide with each other.

When the lens capsule S is in the distance vision state, the state shown in the upper part of FIG. 12 is changed to the state shown in the lower part of FIG. 12. The state shown in the upper part of FIG. 12 is a state in which the ciliary muscle Cm of the ciliary body C contracts and protrudes radially inward in a centripetal direction, and the degree of tension of the Zinn's zonule Z is reduced (in a near vision state). The state shown in the lower part of FIG. 12 is a state in which the ciliary muscle Cm of the ciliary body C is relaxed so that the ciliary body C is positioned radially outward, pulling the lens capsule S via the Zinn's zonule Z, and therefore the degree of tension of the Zinn's zonule Z has been increased. Therefore, the tension of the peripheral part of the equator Se of the lens capsule S increases, thereby increasing the pressing force to the anterior bag portion 11 and the posterior bag portion 12 by the anterior capsule Sf and the posterior capsule Sb, which causes the anterior capsule Sf to move backward while expanding the lens capsule S in the radial direction.

At this time, when the anterior support portion 23 of the intraocular lens 2 receives a pressing force radially inward and rearward from the anterior bag portion 11, the anterior support portion 23 is moved radially inward and rearward by being pushed by the anterior bag portion 11 with the base end portion 23b of the anterior support portion 23 serving as a fulcrum. At this time, the anterior end portion 23a of the anterior support portion 23 in the lens capsule S maintains the radial position and the anterior-posterior position, and the base end portion 23b of the anterior support portion 23 maintains the radial position by the optical portion 21. Therefore, the entire anterior support portion 23 becomes a state of being greatly deflected radially inward and rearward, causing the anterior end portion 23a of the anterior support portion 23 to be moved rearward by an amount larger than the movement of the anterior capsule Sf. In accordance with the movement, the optical portion 21 can also greatly move rearward.

On the other hand, when the lens capsule S is in the near vision state, the state shown in the lower part of FIG. 12 is changed to the state shown in the upper part of FIG. 12. The state shown in the lower part of FIG. 12 is a state in which the ciliary muscle Cm of the ciliary body C is relaxed so that the ciliary body C is positioned radially outward, and the degree of tension of the Zinn's zonule Z is increased (distance vision state) by pulling the lens capsule S via the Zinn's zonule Z. The state shown in the upper part of FIG. 12 is a condition in which the ciliary muscle Cm of the ciliary body C is contracted and protrudes radially inward in the centripetal direction, and the degree of tension of the Zinn's zonule Z is reduced. Therefore, since the tension of the peripheral part of the equator Se of the lens capsule S is relaxed, the pressing force to the anterior support portion 23 and the posterior support portion 24 by the anterior capsule Sf and the posterior capsule Sb is reduced.

At this time, the anterior support portion 23 of the intraocular lens 2 moves radially outward and forward so as to push back the anterior bag portion 11 and the anterior capsule Sf of the lens capsule expansion bag 1 by the elastic force of the anterior support portion 23 with the base end portion 23b of the anterior support portion 23 serving as a fulcrum. At this time, the base end portion 23b of the anterior support portion 23 in the lens capsule S maintains the radial position and the anterior-posterior direction position, and the anterior end portion 23a of the anterior support portion 23 maintains the radial position. Therefore, the anterior support portion 23 returns greatly to the original state (the near vision state shown in the upper part of FIG. 12) in the radially outward and forward direction. With this, the anterior end portion 23a of the anterior support portion 23 is moved forward by an amount larger than the movement of the anterior capsule Sf. In accordance with the movement, the optical portion 21 can be largely moved forward.

In this respect, in a conventional accommodating intraocular lens, when the lens capsule S is in a distance vision or in a near vision state, it is not configured such that the slight movements of the lens capsule S are not amplified to the movements of the optical portion. That is, when the anterior capsule Sf increases or decreases in the radially inward and rearward pressing force to the support portion, the entire support portion expands or narrows radially, and the slights movements of the lens capsule S are merely transmitted to the optical portion as they are. For this reason, the optical portion cannot be moved backward or forward greatly, which cannot exert a practical focus adjustment function.

However, in the intraocular lens 2, as described above, the anterior end portion 23a of the anterior support portion 23 is moved in the anterior-posterior direction by an amount larger than the movement of the anterior capsule Sf. In accordance with the movement, the optical portion 21 is also largely moved in the anterior-posterior direction. For this reason, the slight movements of the lens capsule S can be captured and amplified to large movements of the optical portion 21 in the anterior-posterior direction, which enables to exert a practical focus adjustment function.

According to the calculation on the drawing in this embodiment, the intraocular lens 2 has an amplifying function of 2 times to 2.5 times. Thus, the movements of the anterior capsule Sf of 0.25 mm is amplified to the movements of the optical portion 21 of 0.5 mm to 0.625 mm. When the ability of the optical portion 21 is 22 diopters, a practical adjustment ability of 1.0 diopters to 1.25 diopters can be obtained.

Particularly, in this embodiment, when the lens capsule S is in the near vision state, the anterior end portion 23a of the anterior support portion 23 is positioned forward of the center position of the equator Se of the lens capsule S. On the other hand, when the lens capsule S is in the distance vision state, the anterior end portion 23a is positioned rearward of the center position of equator Se of the lens capsule S. Therefore, the optical portion 21 more largely moves rearward or forward, and therefore the focus adjustment function can be expressed more effectively.

In addition, when the lens capsule S is in a distance vision, the intermediate bag portion 13 of the lens capsule expansion bag 1 deforms so as to expand radially outward in accordance with the movement of the equator Se of the lens capsule S while contacting the equator Se of the lens capsule S. On the other hand, when the lens capsule S is in a near vision, the intermediate bag portion 13 of the lens capsule expansion bag 1 deforms so as to contract radially inward in accordance with the movement of the equator Se of the lens capsule S while contacting the equator Se of the lens capsule S. Therefore, it becomes a state in which there is always no gap between the lens capsule expansion bag 1 and the equator Se of the lens capsule S and therefore the equator Se of the lens capsule S is always open in the anterior-posterior direction. Thus, it is possible to assuredly prevent the adhesion of the lens capsule S and the subsequent hardening of the lens capsule S due to the proliferation and fibrosis of lens epithelial cells.

Note that in this embodiment, the radial position and the anterior-posterior direction positions of the base end portion 23b of the anterior support portion 23 are completely maintained. However, it should be understood that the state in which the base end portion 23b of the anterior support portion 23 maintains the radial direction position includes the state in which it moves in the radial direction within 10% of the entire diameter of the intraocular lens 2. It is also be understood that the state in which the base end portion 23b of the anterior support portion 23 maintains the position in the anterior-posterior direction includes not only the state in which the position in the anterior-posterior direction is completely maintained but also the state in which it moves in the anterior-posterior direction within 10% of the total height of the intraocular lens 2 in the anterior-posterior direction. Furthermore, it should be understood that the condition in which the anterior end portion 23a of the anterior support portion 23 maintains the radial position includes not only the state in which the radial position is completely maintained but also the state in which it moves in the radial direction within 8% of the total diameter of the intraocular lens 2.

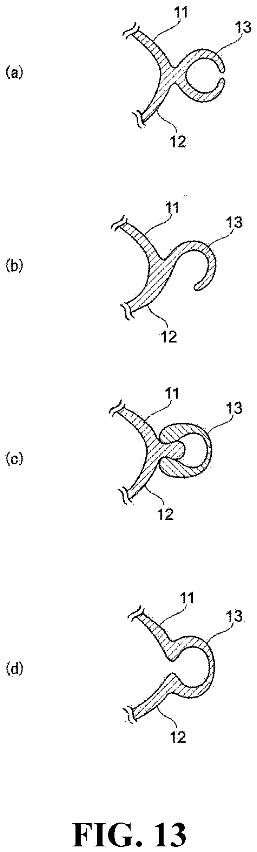

In the lens capsule expansion bag 1, the shapes and the structures of the anterior bag portion 11, the posterior bag portion 12, and the intermediate bag portion 13 are not limited to the shapes and structures described above. For example, various shapes and structures as shown in FIG. 13 are exemplified. They may be formed in a C-shaped shape in cross-section ((a) of FIG. 13). They may be formed in a hook shape in cross-section ((b) of FIG. 13). They may be those formed separately ((c) of FIG. 13). The connecting portion between the anterior bag portion 11 and the posterior bag portion 12 may be formed to be thick ((d) of FIG. 13).

The lens capsule expansion bag 1 is provided with the openings 111, 112, 121, 122, 131, 132 formed in the anterior bag portion 11, the posterior bag portion 12, and the intermediate bag portion 13, respectively, but the opening thereof may not be formed.

Further, although the intraocular lens 2 is provided with six support portions 22, it may be provided with another number of support portions 22

Further, in the intraocular lens 2, an optical portion different from the optical portion 21 may be fitted in the inner peripheral portion of the posterior ring portion 26.

### <Second Embodiment>

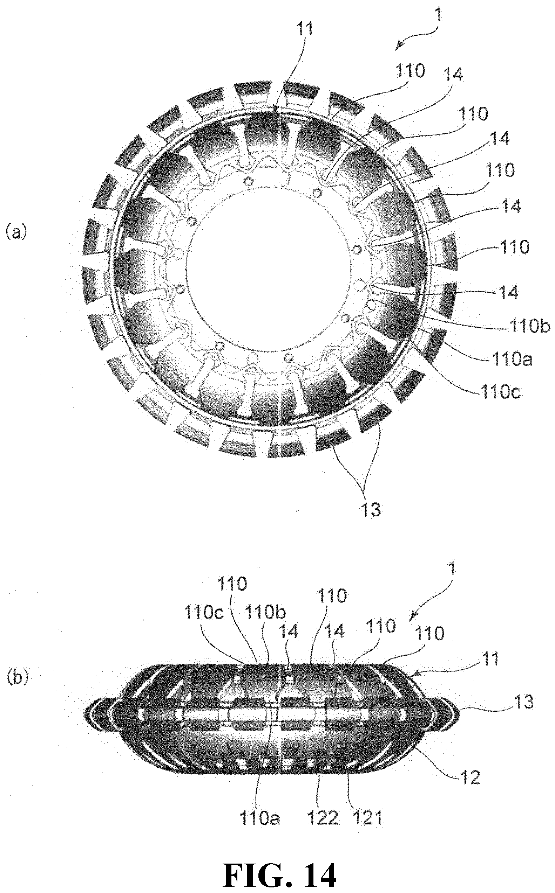

Next, a second embodiment of an accommodating intraocular lens according to the present invention will be described with reference to FIG. 14.

The anterior bag portion 11 is composed of 16 anterior bag pieces 110 provided along the circumferential direction. These anterior bag pieces 110 are formed in the same shape. The width in the circumferential direction is narrowed at the outer peripheral portion 110a, gradually widened in the direction from the outer peripheral portion 110a to the central portion 110c, and slightly narrowed in the direction from the central portion 110c to the inner peripheral portion 110b.

Further, the anterior bag pieces 110 are connected by a deformable strip-shaped connecting portion 14 in the circumferential direction at the inner peripheral portions 110b of the adjacent anterior bag pieces 110 to each other. In this embodiment, the connecting portion 14 is formed in a mountain shape protruding radially inward of the lens capsule expansion bag 1.

This makes it easier for the anterior bag portion 11 to deform rearward and forward, so that the change in the pressing force with respect to the anterior bag portion 11 of the lens capsule expansion bag 1 by the anterior capsule Sf can be more accurately transmitted by the anterior support portion 23 of the intraocular lens 2.

Note that in this embodiment, an example is shown in which the connecting portion 14 is formed in a mountain shape protruding radially inward, but it may be formed in a mountain shape protruding radially outward. Further, it should be noted that it is enough that the connecting portion 14 is deformable in response to the pressing force from the lens capsule. The connecting portion 14 may be deformed from a straight shape to a mountain shape in response to the pressing force from the lens capsule or may be linearly expanded and contracted in the circumferential direction.

The embodiments of the present invention have been described above with reference to the attached drawings, but the present invention is not limited to the illustrated embodiments. It should be understood that various modifications and variations can be made to the illustrated embodiments falling within the same scope as the present invention.

### Description of Symbols

- 1:: Lens capsule expansion bag
11: Anterior bag portion
11a: Outer peripheral portion
11b: Inner peripheral portion
11c; Insertion opening
110: Anterior bag piece
110a: Outer peripheral portion
110b: Inner peripheral portion
111: Linear opening
112: Oval-shaped opening
12: Posterior bag portion
12a: Outer peripheral portion
12b: Inner peripheral portion
121: Linear opening
122: Oval-shaped opening
13: Intermediate bag portion
131: Linear opening
132: Circular opening
14: Connecting portion
- 2:: Intraocular lens
21: Optical portion
22: Support portion
23: Anterior support portion
23a: Anterior end portion
23b: Base end portion
24: Posterior support portion
24a: Posterior end portion
24b: Base end portion
25: Anterior ring portion
25a: Groove
26: Posterior ring portion
27: Restriction portion
27a: Anterior end portion

## Claims

1. An accommodating intraocular lens comprising:
a lens capsule expansion bag (1) to be placed in the lens capsule (S) whose anterior capsule (Sf) has been incised in ophthalmic surgery; and
an intraocular lens (2) placed in the lens capsule expansion bag (1),
wherein the lens capsule expansion bag (1) has an elastic force,
wherein the lens capsule expansion bag (1) includes:
an annular anterior bag portion (11) adapted to be placed in such a manner as to be in contact with an anterior capsule (Sf) of the lens capsule (S);
an annular posterior bag portion (12) adapted to be placed in such a manner as to be in contact with a posterior capsule (Sb) of the lens capsule (S); and
an annular intermediate bag portion (13) protruding radially outward from an outer peripheral portion (11a) of the anterior bag portion (11) and an outer peripheral portion (12a) of the posterior bag portion (12), the annular intermediate bag portion (13) being configured to be placed in such a manner as to be in contact with an equator (Se) of the lens capsule (S),
wherein the intraocular lens (2) includes:
an optical portion (21); and
one or a plurality of support portions (22) arranged around the optical portion (21) to support the optical portion (21),
wherein the support portion (22) has an elastic force,
wherein the support portion (22) includes:
an anterior support portion (23) placed in such a manner as to be in contact with an inner surface of the anterior bag portion (11); and
a posterior support portion (24) placed in such a manner as to be in contact with an inner surface of the posterior bag portion (12),
wherein the anterior support portion (23) extends radially inward and forward from a base end portion (23b) connected to the posterior support portion (24) and then extends radially inward and rearward, an anterior end portion (23a) of the anterior support portion (23) being connected to a peripheral portion of the optical portion (21),
wherein the accommodating intraocular lens is adapted such when the lens capsule (S) is in a distance vision state, as a pressing force by the anterior bag portion (11) to the anterior support portion (23) of the intraocular lens (2) increases in accordance with an increase in a pressing force of the lens capsule expansion bag (1) by the anterior capsule (Sf) to the anterior support portion (23) of the lens capsule expansion bag (1),
the anterior support portion (23) deflects radially inward and rearward while maintaining a radial position of the base end portion (23b) and a radial position of the anterior end portion (23a), causing a rearward movement of the anterior end portion (23a) of the anterior support portion (23) while maintaining the radial position of the anterior end portion (23a) of the anterior support portion (23), which moves the optical portion (21) rearward, and
the intermediate bag portion (13) of the lens capsule expansion bag (1) deforms to expand radially outward in response to a movement of the equator (Se) of the lens capsule (S) while contacting the equator (Se) of the lens capsule (S), and
wherein the accommodating intraocular lens is adapted such when the lens capsule (S) is in a near vision state, as a pressing force by the anterior bag portion (11) to the anterior support portion (23) of the intraocular lens (2) decreases in accordance with a decrease in a pressing force of the lens capsule expansion bag (1) by the anterior capsule (Sf) to the anterior support portion (23) of the lens capsule expansion bag (1),
the anterior support portion (23) deflects radially outward and forward while maintaining a radial position of the base end portion (23b) and a radial position of the anterior end portion (23a), causing a forward movement of the anterior end portion (23a) of the anterior support portion (23) while maintaining the radial position of the anterior end portion (23a) of the anterior support portion (23), which moves the optical portion (21) forward, and
the intermediate bag portion (13) of the lens capsule expansion bag (1) deforms to contract radially inward in response to a movement of the equator (Se) of the lens capsule (S) while contacting the equator (Se) of the lens capsule (S).

2. The accommodating intraocular lens as recited in claim 1,
wherein the lens capsule expansion bag (1) is configured such that the intermediate bag portion (13) presses the equator (Se) of the lens capsule (S),
wherein the accommodating intraocular lens is adapted such when the lens capsule (S) is in the distance vision state, the intermediate bag portion (13) deforms to expand radially outward in response to the movement of the equator (Se) of the lens capsule (S) while pressing the equator (Se) of the lens capsule (S), and
wherein the accommodating intraocular lens is adapted such when the lens capsule (S) is in the near vision state, the intermediate bag portion (13) deforms to contract radially inward in response to the movement of the equator (Se) of the lens capsule (S) while pressing the equator (Se) of the lens capsule (S).

3. The accommodating intraocular lens as recited in claim 1,
wherein the lens capsule expansion bag (1) is configured such that the anterior bag portion (11), the posterior bag portion (12), and the intermediate bag portion (13) are continuously formed of a flexible membrane-like or a plate-like elastic member.

4. The accommodating intraocular lens (2) as recited in claim 1,
wherein an inner peripheral portion (11b) of at least one of the anterior bag portion (11) and the posterior bag portion (12) of the lens capsule expansion bag (1) is formed to be thicker than a portion of the lens capsule expansion bag (1) other than the inner peripheral portion (11b).

5. The accommodating intraocular lens as recited in claim 1,
wherein the lens capsule expansion bag (1) is configured such that the intermediate bag portion (13) is smaller in rigidity than the anterior bag portion (11) and the posterior bag portion (12).

6. The accommodating intraocular lens as recited in claim 1,
wherein the lens capsule expansion bag (1) is provided with a plurality of openings formed in a side surface of the intermediate bag portion (13) at predetermined intervals.

7. The accommodating intraocular lens as recited in claim 1,
wherein the lens capsule expansion bag (1) is coated with an MPC-polymer on at least one of an outer surface and a back surface.

8. The accommodating intraocular lens as recited in claim 1,
wherein the lens capsule expansion bag (1) is formed of a gel-like material with lubricity

9. The accommodating intraocular lens as recited in claim 1,
wherein the anterior bag portion (11) is composed of a plurality of anterior bag pieces (110) arranged along a circumferential direction, and
wherein adjacent anterior bag pieces (110) are connected in a circumferential direction by a deformable connecting portion (14).

10. The accommodating intraocular lens as recited in claim 9,
wherein the connecting portion (14) is formed in a mountain shape protruding radially inward or radially outward of the lens capsule expansion bag (1).

## Patentansprüche

1. Eine anpassbare Intraokularlinse, die aufweist:
eine Linsenkapsel-Expansionstasche (1), die in die Linsenkapsel (S) eingesetzt werden soll, deren vordere Kapsel (Sf) in einer Augenoperation eingeschnitten wurde, und
eine Intraokularlinse (2), die in die Linsenkapsel-Expansionstasche (1) eingesetzt ist,
wobei die Linsenkapsel-Expansionstasche (1) eine elastische Kraft hat,
wobei die Linsenkapsel-Expansionstasche (1) aufweist:
einen ringförmigen vorderen Taschenabschnitt (11), der dazu geeignet ist, auf eine Weise angeordnet zu werden, um mit einer vorderen Kapsel (Sf) der Linsenkapsel (S) in Kontakt zu sein,
einen ringförmigen hinteren Taschenabschnitt (12), der dazu geeignet ist, auf eine Weise angeordnet zu werden, um mit einer hinteren Kapsel (Sb) der Linsenkapsel (S) in Kontakt zu sein, und
einen ringförmigen mittleren Abschnitt (13), der radial nach außen von einem Außenrandabschnitt (11a) des vorderen Taschenabschnitts (11) und einem Außenrandabschnitt (12a) des hinteren Taschenabschnitts (12) vorsteht, wobei der ringförmige mittlere Taschenabschnitt (13) konfiguriert ist, um auf eine Weise angeordnet zu werden, um mit einem Äquator (Se) der Linsenkapsel (S) in Kontakt zu sein,
wobei die Intraokularlinse (2) aufweist:
einen optischen Abschnitt (21) und
einen oder eine Mehrzahl von Stützabschnitten (22), die um den optischen Abschnitt (21) herum angeordnet sind, um den optischen Abschnitt (21) zu stützen,
wobei der Stützabschnitt (22) eine elastische Kraft hat,
wobei der Stützabschnitt (22) aufweist:
einen vorderen Stützabschnitt (23), der auf eine Weise angeordnet ist, um mit einer Innenfläche des vorderen Taschenabschnitts (11) in Kontakt zu sein, und
einen hinteren Stützabschnitt (24), der auf eine Weise angeordnet ist, um mit einer Innenfläche des hinteren Taschenabschnitts (12) in Kontakt zu sein,
wobei sich der vordere Stützabschnitt (23) von einem mit dem hinteren Stützabschnitt (24) verbundenen Basisendabschnitt (23b) radial nach innen und vorne erstreckt und sich dann radial nach innen und hinten erstreckt, wobei ein vorderer Endabschnitt (23a) des vorderen Stützabschnitts (23) mit einem Randabschnitt des optischen Abschnitts (21) verbunden ist,
wobei die anpassbare Intraokularlense geeignet ist, dass, wenn die Linsenkapsel (S) in einem Weitsichtzustand ist, wenn eine Druckkraft mittels des vorderen Taschenabschnitts (11) auf den vorderen Stützabschnitt (23) der Intraokularlinse (2) erhöht wird gemäß einer Erhöhung einer Druckkraft der Linsenkapsel-Expansionstasche (1) mittels der vorderen Kapsel (Sf) auf den vorderen Stützabschnitt (23) der Linsenkapsel-Expansionstasche (1),
der vordere Stützabschnitt (23) radial nach innen und hinten auslenkt, während er eine radiale Position des Basisendabschnitts (23b) und eine radiale Position des vorderen Endabschnitts (23a) beibehält, wodurch eine Rückwärtsbewegung des vorderen Endabschnitts (23a) des vorderen Stützabschnitts (23) verursacht wird, während die radiale Position des vorderen Endabschnitts (23a) des vorderen Stützabschnitts (23) beibehalten wird, wodurch der optische Abschnitt (21) sich nach hinten bewegt, und
der mittlere Taschenabschnitt (13) der Linsenkapsel-Expansionstasche (1) sich verformt, um sich radial nach außen auszudehnen in Antwort auf eine Bewegung des Äquators (Se) der Linsenkapsel (S), während er mit dem Äquator (Se) der Linsenkapsel (S) in Kontakt ist, und
wobei die anpassbare Intraokularlense geeignet ist, dass, wenn die Linsenkapsel (S) in einem Kurzsichtzustand ist, wenn eine Druckkraft mittels des vorderen Taschenabschnitts (11) auf den vorderen Stützabschnitt (23) der Intraokularlinse (2) verringert wird gemäß einer Verringerung einer Druckkraft der Linsenkapsel-Expansionstache (1) mittels der vorderen Kapsel (Sf) auf den vorderen Stützabschnitt (23) der Linsenkapsel-Expansionstasche (1),
der vordere Stützabschnitt (23) radial nach außen und nach vorne auslenkt, während er eine radiale Position des Basisendabschnitts (23b) und eine radiale Position des vorderen Stützabschnitts (23a) beibehält, wodurch eine Vorwärtsbewegung des vorderen Endabschnitts (23a) des vorderen Stützabschnitts (23) verursacht wird, während die radiale Position des vorderen Endabschnitts (23a) des vorderen Stützabschnitts (23) beibehalten wird, wodurch der optische Abschnitt (21) sich nach vorne bewegt, und
der mittlere Taschenabschnitt (13) der Linsenkapsel-Expansionstasche (1) sich verformt, um sich radial nach innen zusammenzuziehen in Antwort auf eine Bewegung des Äquators (Se) der Linsenkapsel (S), während er mit dem Äquator (Se) der Linsenkapsel (S) in Kontakt ist.

2. Die anpassbare Intraokularlinse gemäß Anspruch 1,
wobei die Linsenkapsel-Expansionstasche (1) konfiguriert ist, sodass der mittlere Taschenabschnitt (13) auf den Äquator (Se) der Linsenkapsel (S) drückt,
wobei die anpassbare Intraokularlinse geeignet ist, dass, wenn die Linsenkapsel (S) im Weitsichtzustand ist, der mittlere Taschenabschnitt (13) sich verformt, um sich radial nach außen auszudehnen in Antwort auf die Bewegung des Äquators (Se) der Linsenkapsel (S), während er den Äquator (Se) der Linsenkapsel (S) drückt, und
wobei die anpassbare Intraokularlinse geeignet ist, dass, wenn die Linsenkapsel (S) im Kurzsichtzustand ist, der mittlere Taschenabschnitt (13) sich verformt, um sich radial nach innen zusammenzuziehen in Antwort auf die Bewegung des Äquators (Se) der Linsenkapsel (S), während er den Äquator (Se) der Linsenkapsel (S) drückt.

3. Die anpassbare Intraokularlinse gemäß Anspruch 1,
wobei die Linsenkapsel-Expansionstasche (1) konfiguriert ist, sodass der vordere Taschenabschnitt (11), der hintere Taschenabschnitt (12) und der mittlere Taschenabschnitt (13) kontinuierlich aus einem flexiblen membranartigen oder einem plattenartigen elastischen Element gebildet sind.

4. Die anpassbare Intraokularlinse (2) gemäß Anspruch 1,
wobei ein innerer Randbereich (11b) von mindestens einem von dem vorderen Taschenabschnitt (11) und dem hinteren Taschenabschnitt (12) der Linsenkapsel-Expansionstasche (1) ausgebildet ist, um dicker zu sein als ein anderer Abschnitt der Linsenkapsel-Expansionstasche (1) als der innere Randbereich (11b).

5. Die anpassbare Intraokularlinse gemäß Anspruch 1,
wobei die Linsenkapsel-Expansionstasche (1) konfiguriert ist, sodass der mittlere Taschenabschnitt (13) eine geringere Steifigkeit aufweist als der vordere Taschenabschnitt (11) und der hintere Taschenabschnitt (12).

6. Die anpassbare Intraokularlinse gemäß Anspruch 1,
wobei die Linsenkapsel-Expansionstasche (1) mit einer Mehrzahl von Öffnungen bereitgestellt ist, die in einer Seitenfläche des mittleren Taschenabschnitts (13) in vorbestimmten Intervallen ausgebildet sind.

7. Die anpassbare Intraokularlinse gemäß Anspruch 1,
wobei die Linsenkapsel-Expansionstasche (1) auf mindestens einer von einer Außenfläche und einer Rückfläche mit einem MPC-Polymer beschichtet ist.

8. Die anpassbare Intraokularlinse gemäß Anspruch 1,
wobei die Linsenkapsel-Expansionstasche (1) aus einem gelartigen Material mit Gleitfähigkeit gebildet ist.

9. Die anpassbare Intraokularlinse gemäß Anspruch 1,
wobei der vordere Taschenabschnitt (11) aus einer Mehrzahl von vorderen Taschenteilen (110) gebildet ist, die entlang einer Umfangsrichtung angeordnet sind, und
wobei benachbarte vordere Taschenteile (110) in einer Umfangsrichtung mittels eines verformbaren Verbindungsabschnitts (14) verbunden sind.

10. Die anpassbare Intraokularlinse gemäß Anspruch 9,
wobei der Verbindungsabschnitt (14) in einer Bergform ausgebildet ist, die radial nach innen oder radial nach außen von der Linsenkapsel-Expansionstasche (1) vorsteht.

## Revendications

1. Lentille intraoculaire accommodative, comprenant :
un sac d'expansion de capsule de lentille (1) destiné à être placé dans la capsule de lentille (S) dont la capsule antérieure (Sf) a été incisée lors d'une chirurgie ophtalmique ; et
une lentille intraoculaire (2) placée dans le sac d'expansion de capsule de lentille (1),
dans laquelle le sac d'expansion de capsule de lentille (1) a une force élastique,
dans laquelle le sac d'expansion de capsule de lentille (1) comprend :
une partie de sac antérieure annulaire (11) adaptée pour être placée de manière à être en contact avec une capsule antérieure (Sf) de la capsule de lentille (S) ;
une partie de sac postérieure annulaire (12) adaptée pour être placée de manière à être en contact avec une capsule postérieure (Sb) de la capsule de lentille (S) ; et
une partie de sac intermédiaire annulaire (13) faisant saillie radialement vers l'extérieur à partir d'une partie périphérique extérieure (11a) de la partie de sac antérieure (11) et d'une partie périphérique extérieure (12a) de la partie de sac postérieure (12), la partie de sac intermédiaire annulaire (13) étant configurée pour être placée de manière à être en contact avec un équateur (Se) de la capsule de lentille (S),
dans laquelle la lentille intraoculaire (2) comprend :
une partie optique (21) ; et
une ou une pluralité de parties de support (22) disposées autour de la partie optique (21) pour supporter la partie optique (21),
dans laquelle la partie de support (22) a une force élastique,
dans laquelle la partie de support (22) comprend :
une partie de support antérieure (23) placée de manière à être en contact avec une surface intérieure de la partie de sac antérieure (11) ; et
une partie de support postérieure (24) placée de manière à être en contact avec une surface intérieure de la partie de sac postérieure (12),
dans laquelle la partie de support antérieure (23) s'étend radialement vers l'intérieur et vers l'avant à partir d'une partie d'extrémité de base (23b) reliée à la partie de support postérieure (24), puis s'étend radialement vers l'intérieur et vers l'arrière, une partie d'extrémité antérieure (23a) de la partie de support antérieure (23) étant reliée à une partie périphérique de la partie optique (21),
dans laquelle la lentille intraoculaire accommodative est adaptée de telle sorte que, lorsque la capsule de lentille (S) est dans un état de vision de loin, lorsqu'une force de pression par la partie de sac antérieure (11) sur la partie de support antérieure (23) de la lentille intraoculaire (2) augmente en fonction d'une augmentation d'une force de pression du sac d'expansion de capsule de lentille (1) par la capsule antérieure (Sf) sur la partie de support antérieure (23) du sac d'expansion de capsule de lentille (1),
la partie de support antérieure (23) fléchit radialement vers l'intérieur et vers l'arrière tout en maintenant une position radiale de la partie d'extrémité de base (23b) et une position radiale de la partie d'extrémité antérieure (23a), provoquant un mouvement vers l'arrière de la partie d'extrémité antérieure (23a) de la partie de support antérieure (23) tout en maintenant la position radiale de la partie d'extrémité antérieure (23a) de la partie de support antérieure (23), ce qui déplace la partie optique (21) vers l'arrière, et
la partie de sac intermédiaire (13) du sac d'expansion de capsule de lentille (1) se déforme pour s'étendre radialement vers l'extérieur en réponse à un mouvement de l'équateur (Se) de la capsule de lentille (S) tout en contactant l'équateur (Se) de la capsule de lentille (S), et
dans laquelle la lentille intraoculaire accommodative est adaptée de telle sorte que, lorsque la capsule de lentille (S) est dans un état de vision de près, lorsqu'une force de pression par la partie de sac antérieure (11) sur la partie de support antérieure (23) de la lentille intraoculaire (2) diminue en fonction d'une diminution d'une force de pression du sac d'expansion de capsule de lentille (1) par la capsule antérieure (Sf) sur la partie de support antérieure (23) du sac d'expansion de capsule de lentille (1),
la partie de support antérieure (23) fléchit radialement vers l'extérieur et vers l'avant tout en maintenant une position radiale de la partie d'extrémité de base (23b) et une position radiale de la partie d'extrémité antérieure (23a), provoquant un mouvement vers l'avant de la partie d'extrémité antérieure (23a) de la partie de support antérieure (23) tout en maintenant la position radiale de la partie d'extrémité antérieure (23a) de la partie de support antérieure (23), ce qui déplace la partie optique (21) vers l'avant, et
la partie de sac intermédiaire (13) du sac d'expansion de capsule de lentille (1) se déforme pour se contracter radialement vers l'intérieur en réponse à un mouvement de l'équateur (Se) de la capsule de lentille (S) tout en contactant l'équateur (Se) de la capsule de lentille (S).

2. Lentille intraoculaire accommodative selon la revendication 1,
dans laquelle le sac d'expansion de capsule de lentille (1) est configuré de telle sorte que la partie de sac intermédiaire (13) appuie sur l'équateur (Se) de la capsule de lentille (S),
dans laquelle la lentille intraoculaire accommodative est adaptée de telle sorte que lorsque la capsule de lentille (S) est dans l'état de vision de loin, la partie de sac intermédiaire (13) se déforme pour s'étendre radialement vers l'extérieur en réponse au mouvement de l'équateur (Se) de la capsule de lentille (S) tout en appuyant sur l'équateur (Se) de la capsule de lentille (S), et
dans laquelle la lentille intraoculaire accommodative est adaptée de telle sorte que, lorsque la capsule de lentille (S) est dans l'état de vision de près, la partie de sac intermédiaire (13) se déforme pour se contracter radialement vers l'intérieur en réponse au mouvement de l'équateur (Se) de la capsule de lentille (S) tout en appuyant sur l'équateur (Se) de la capsule de lentille (S).

3. Lentille intraoculaire accommodative selon la revendication 1,
dans laquelle le sac d'expansion de capsule de lentille (1) est configuré de telle sorte que la partie de sac antérieure (11), la partie de sac postérieure (12) et la partie de sac intermédiaire (13) sont formées de manière continue d'un élément élastique flexible de type membrane ou de type plaque.

4. Lentille intraoculaire accommodative (2) selon la revendication 1,
dans laquelle une partie périphérique intérieure (11b) d'au moins l'une parmi la partie de sac antérieure (11) et la partie de sac postérieure (12) du sac d'expansion de capsule de lentille (1) est formée de manière à être plus épaisse qu'une partie de sac d'expansion de capsule de lentille (1) autre que la partie périphérique intérieure (11b).

5. Lentille intraoculaire accommodative selon la revendication 1,
dans laquelle le sac d'expansion de capsule de lentille (1) est configuré de telle sorte que la partie de sac intermédiaire (13) présente une rigidité inférieure à celle de la partie de sac antérieure (11) et de la partie de sac postérieure (12).

6. Lentille intraoculaire accommodative selon la revendication 1,
dans laquelle le sac d'expansion de capsule de lentille (1) est muni d'une pluralité d'ouvertures formées dans une surface latérale de la partie de sac intermédiaire (13) à des intervalles prédéterminés.

7. Lentille intraoculaire accommodative selon la revendication 1,
dans laquelle le sac d'expansion de capsule de lentille (1) est recouvert d'un polymère MPC sur au moins l'une d'une surface extérieure et d'une surface arrière.

8. Lentille intraoculaire accommodative selon la revendication 1,
dans laquelle le sac d'expansion de capsule de lentille (1) est formé d'un matériau de type gel présentant des propriétés lubrifiantes.

9. Lentille intraoculaire accommodative selon la revendication 1,
dans laquelle la partie de sac antérieure (11) est composée d'une pluralité de morceaux de sac antérieurs (110) disposés le long d'une direction circonférentielle, et
dans laquelle des morceaux de sac antérieurs adjacents (110) sont reliés dans une direction circonférentielle par une partie de connexion déformable (14).

10. Lentille intraoculaire accommodative selon la revendication 9,
dans laquelle la partie de connexion (14) est formée en une forme de montagne faisant saillie radialement vers l'intérieur ou radialement vers l'extérieur du sac d'expansion de capsule de lentille (1).
